# EUROPÄISCHE PATENTSCHRIFT

(11) **EP 1 411 838 B1**
(45) Veröffentlichungstag und Bekanntmachung des Hinweises auf die Patenterteilung: **17.10.2007**
(21) Anmeldenummer: 02700223.7
(22) Anmeldetag: 12.02.2002
(51) Int. Cl.: A61B 17/06

(54) **INSTRUMENTARIUM ZUR OPERATIVEN BEHANDLUNG DER HARNINKONTINENZ DER FRAU**
INSTRUMENT FOR SURGICALLY TREATING FEMALE URINARY INCONTINENCE
INSTRUMENT DE TRAITEMENT CHIRURGICAL DE L'INCONTINENCE URINAIRE CHEZ LA FEMME

(30) Priorität: 17.02.2001 DE 10107520
(43) Veröffentlichungstag der Anmeldung: 28.04.2004
(73) Patentinhaber: Riek, Siegfried, Dr. med., D-78628 Rottweil (DE); Gaiselmann, Thomas, D-78667 Villingendorf (DE); Bachmann, Karl-Heinz, 78667 Villingendorf (DE); Marzusch, Klaus, 72072 Tübingen (DE); Walwiener, Diethelm, 72076 Tübingen (DE)
(72) Erfinder: Riek, Siegfried, Dr. med., D-78628 Rottweil (DE); Gaiselmann, Thomas, D-78667 Villingendorf (DE); Bachmann, Karl-Heinz, 78667 Villingendorf (DE); Marzusch, Klaus, 72072 Tübingen (DE); Walwiener, Diethelm, 72076 Tübingen (DE)
(74) Vertreter: Patentanwälte Westphal, Mussgnug & Partner
(86) Internationale Anmeldenummer: PCT/EP2002/001427
(87) Internationale Veröffentlichungsnummer: WO 2002/065923

(56) Entgegenhaltungen:
- EP-A- 1 093 758
- WO-A-00/74613
- WO-A-97/13465
- GB-A- 445 656
- GB-A- 670 199
- US-A- 5 899 909

## Beschreibung

Die Erfindung betrifft ein Instrumentarium zur operativen Behandlung der Harninkontinenz der Frau.

Die Harninkontinenz der Frau entsteht häufig durch Bindegewebsschwäche, die sich mit unterschiedlicher Intensität an den Ligg. Pubourethralia, an den seitlichen Verbindungen an der vorderen Vaginalwand mit der Levatormuskulatur und im Septum urethrovesikovaginale manifestiert. Zur Behandlung dieser Harninkontinenz wurde eine Operationstechnik entwickelt, bei welcher ein implantierbares Band eingezogen wird, welches zur Gewebefestigung als Gewebeersatz und zur Bildung von Bindegewebe dient. Diese Operationstechnik ist in der US 5,899,909 beschrieben. Hierbei wird ein Band verwendet, dessen Enden jeweils an einer gebogenen Nadel befestigt sind. Mittels einer medianen Kolpotomie werden die Nadeln von der Vagina ausgehend beiderseits der Urethra an der Schambeinhinterwand aufwärts geführt und nach Incision der Haut durch die Bauchdecke hindurchgestoßen. Das Band legt sich somit als Schleife um die Urethra und wird an den durch die Bauchdecke nach außen geführten Enden positioniert. Die Enden des Bandes mit den daran fest verbundenen Nadeln werden dann in der Subcutis abgeschnitten und die Schleife des Bandes verbleibt im Körper.

Bei dem aus der US 5,899,909 bekannten Instrumentarium wird zum Einführen der gebogenen Nadeln an.deren proximalem Ende ein gerader Schaft mit einem Handgriff lösbar konnektiert. Hierzu wird der Schaft in eine Sackbohrung der proximalen Endstirnfläche der Nadel eingeschraubt. Das Band mit der das Band umschließenden Hülle sind außen auf dem Umfang des proximalem Endes der Nadel befestigt. Das Band mit den an seinen Enden befestigten Nadeln ist ein konfektionierter Einmal-Artikel, während der in die Nadeln einschraubbare Schaft mit dem Griff sterilisierbar und wiederverwendbar ist.

Die Nadeln weisen eine Kreisbogenform auf, die der Kontur der Schambeinhinterwand angepaßt ist, so daß die Nadeln auf einer entsprechenenden Kreisbogenbahn durch das Gewebe gestoßen werden. Der in das proximale Ende der gebogenen Nadel eingesetzte Schaft des Griffes erschwert wegen seiner geradlinigen Ausbildung das Führen der Nadeln auf der Kreisbogenbahn. Die distale Spitze der Nadel ist konisch und symmetrisch zur Mittelachse der Nadel ausgebildet, so daß die Spitze tangential zu dem Kreisbogen der Nadel gerichtet ist. Wird die Nadel an der Schambeinhinterwand hochgeführt, so besteht daher ein gewisses Risiko, daß die Spitze der Nadel die an der Schambeinhinterwand anliegende Harnblase verletzt.

Gemäß der prioritätsälteren nicht vorveröffentlichten EP-A-1 093 758 gehört zum Stand der Technik ein Instrumentarium zur operativen Behandlung der Harninkontinenz der Frau mit einer gebogenen Nadel, deren distales Ende als Spitze zum Penetrieren des Gewebes ausgebildet ist. Die Nadel ist eine Hohlnadel, deren distales Ende abgeschrägt ist, so dass die Spitze durch die in der Krümmungsebene der Nadel deren Krümmungsmittelpunkt zugewandte Mantelfläche der Nadel gebildet wird. Ein implantierbares Band wird durch die hohle Nadel eingeführt und schleifenförmig um die Urethra der Patientin gelegt.

Der Erfindung liegt die Aufgabe zugrunde, das Instrumentarium im Hinblick auf diese geschilderten Nachteile zu verbessern.

Diese Aufgabe wird erfindungsgemäß gelöst durch ein Instrumentarium mit den Merkmalen des Anspruchs 1. Vorteilhafte Ausführungen und Weiterbildungen der Erfindung sind in den Unteransprüchen angegeben.

Beim Einführen der Nadel wird diese mit ihrer perforierenden Spitze an der Schambeinhinterwand entlang zwischen dem Schambein und der Harnblase auf einer der Kontur der Schambeinhinterwand folgenden gebogenen Bahn hindurchgeführt, die dem Krümmungsbogen der Nadel entspricht. Die distale penetrierende Spitze der Nadel ist erfindungsgemäß gegenüber der Mittelachse der Nadel in der Weise versetzt ausgebildet, daß sich die Spitze an der Innenseite des Krümmungsbogens der Nadel befindet. Die Spitze kann auf diese Weise an der Schambeinhinterwand entlanggeführt werden und ragt nicht tangential zu der Bahn der Nadel, so daß die Gefahr einer Verletzung der Harnblasenwand erheblich reduziert ist. Zweckmäßigerweise ist dabei die Spitze in ihrem Scheitelbereich an der Krümmungsinnenseite kufenförmig angeschliffen. Dadurch kann die Spitze der Nadel unmittelbar an der Knochenhaut des Schambeines anliegend geführt werden, wobei der kufenförmige Anschliff der Spitze gewährleistet, daß die Spitze auf der Knochenhaut entlanggleitet, ohne diese verletzen zu können. Dadurch wird die Führung der Nadel beim Durchschieben durch das Gewebe wesentlich leichter und sicherer, da die Schambein-Rückwand mittels der Spitze der Nadel ertastet werden kann, um die Nadel an dieser Schambein-Rückwand entlangzuführen. Begünstigt wird die Führung der Spitze der Nadel an dem Schambein noch dadurch, daß die Spitze gegenüber dem Kreisbogen der Nadel stärker nach innen gebogen ist.

Das Band und die gegebenenfalls das Band umschließende Hülle sind vorzugsweise lösbar an dem proximalen Ende der Nadel befestigt. Dadurch ergibt sich die Möglichkeit, die Nadeln als sterilisierbare Mehrfach-Artikel auszubilden und lediglich das im Körper verbleibende Band als konfektionierten Einmal-Artikel herzustellen. Die Mehrfach-Verwendbarkeit der Nadeln, die vorzugsweise aus hochwertigem Edelstahl gefertigt sind, führt zu einer wesentlichen Kostenreduzierung. Da die Nadeln lösbar mit dem Band verbunden sind, kann gegebenenfalls auch nur eine einzige Nadel verwendet werden, die nacheinander mit den beiden Enden des Bandes konnektiert wird.

Zur Handhabung und Führung der Nadel kann auf diese im proximalen Bereich, dem Endabschnitt, ein Griff aufgesetzt werden. Der Griff umschließt den Endabschnitt der Nadel und hält den Endabschnitt und damit die gesamte Nadel axial fest und unverdrehbar. Damit ist eine bessere Ergonomie bei der Führung der Nadel gegeben. Insbesondere weil der Griff und die Nadel den gleichen Krümmungsradius aufweisen, folgt der Griff in der Verlängerung dem Nadelverlauf und der Perforationsbahn der Nadel im Gewebe. Dadurch ist eine optimale Übertragung des Vorschubdrucks auf die penetrierende Spitze der Nadel gewährleistet. Das Aufsetzen des Griffes außen auf den Endabschnitt der Nadel bedeutet keine Materialschwächung und keine Bruchgefahr durch die Verbindung von Griff und Nadel. Ein seitliches Aufsetzen des Griffes auf die Nadel hat weiter den Vorteil, daß die proximale Endstirnfläche der Nadel nicht zur Konnektierung des Griffes benötigt wird. Das Band und die das Band umschließende Hülle können daher innerhalb des Querschnittes der Nadel gekuppelt werden. Die Kupplung zwischen Nadel bzw. deren Endabschnitt und dem Band und seiner Hülle führt nicht zu einer Querschnittsvergrößerung.

Im folgenden wird die Erfindung anhand von in der Zeichnung dargestellten Ausführungsbeispielen näher erläutert. Es zeigen
- Figur 1: eine Seitenansicht eines Teiles des erfindungsgemäßen Instrumentariums,
- Figur 2: einen Querschnitt gemäß der Linie A-A in Figur 1,
- Figur 3: in vergrößerter perspektivischer Darstellung die Kupplung der Nadel mit dem Band,
- Figur 4: schematisch die an der Schambein-Hinterwand entlanggeführte Spitze der Nadel in einer Seitenansicht senkrecht zur Krümmungsebene der Nadel,
- Figur 5: eine Seitenansicht der Spitze der Nadel in der Krümmungsebene,
- Figur 6: die Spitze in einer abgewandelten Ausführungsform in einem Axialschnitt in der Krümmungsebene,
- Figur 7: eine abgewandelte Ausführung des Instrumentariums in einer Seitenansicht senkrecht zur Krümmungsebene der Nadel,
- Figur 8: eine Seitenansicht dieser Ausführung in der Krümmungsebene der Nadel und
- Figur 9: eine Figur 7 entsprechende Seitenansicht einer weiteren Abwandlung des Instrumentariums.

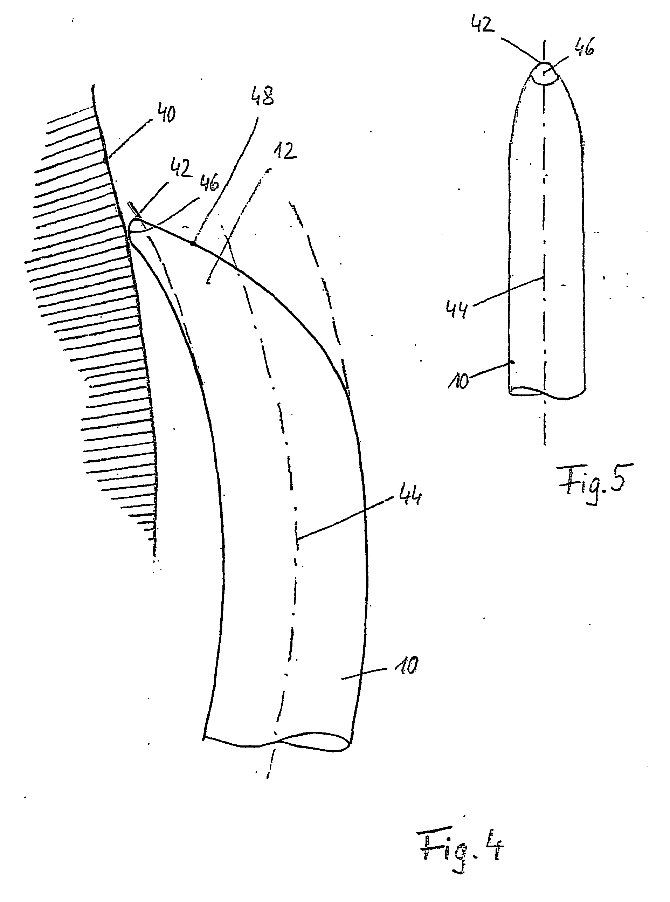

Das Instrumentarium zur operativen Behandlung der Harninkontinenz der Frau weist eine Nadel auf, die aus einem sterilisierbaren Werkstoff, vorzugsweise aus Edelstahl besteht. Die Nadel 10 hat einen kreisförmigen Querschnitt und ist in ihrer Längsrichtung bogenförmig, vorzugsweise kreisbogenförmig gekrümmt. An ihrem distalen Ende weist die Nadel 10 eine Spitze 12 auf, die zum Penetrieren des Gewebes dient und deren Form später anhand der Figuren 4 und 5 näher beschrieben wird.

Die gesamte axiale Länge der Nadel 10 ist so dimensioniert, daß ein distaler vorderer Penetrationsabschnitt gebildet ist, an welchen sich ein proximaler hinterer Endabschnitt 14 anschließt. Die gesamte Nadel 10 bestehend aus vorderem Penetrationsabschnitt und hinterem Endabschnitt 14 ist einstückig mit durchgehend konstantem Querschnitt ausgebildet. Die axiale Länge des vorderen Penetrationsabschnitts ist so dimensioniert, daß die Nadel 10 mit diesem Abschnitt von der Vagina ausgehend bis durch die Bauchdecke hindurchgestoßen werden kann.

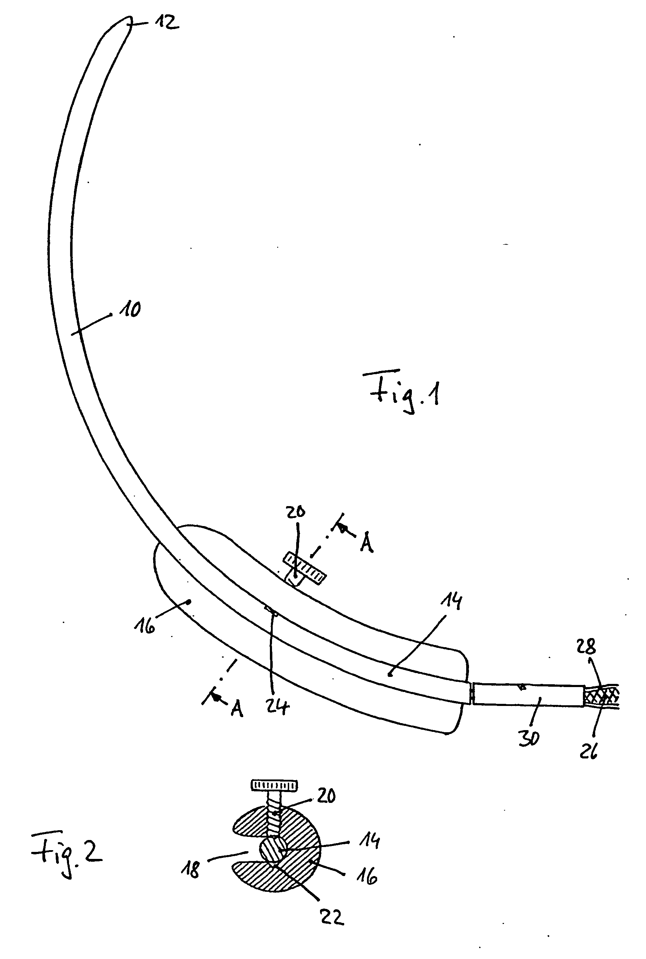

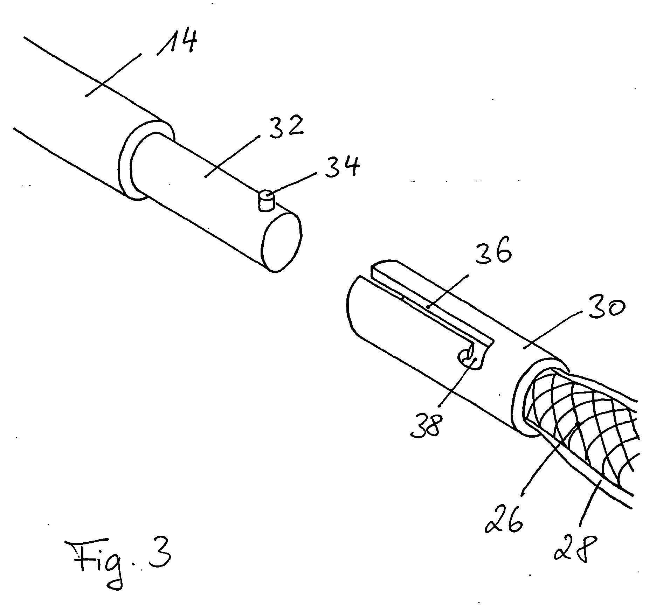

In der Ausführung der Figuren 1 bis 3 ist auf den Endabschnitt 14 ein Griff 16 aufsetzbar, der den Endabschnitt 14 umschließt und eine Länge von beispielsweise 10 cm hat und ergonomisch in der Hand gehalten werden kann. Der Querschnitt des Griffes 16 ist so gewählt, daß er eine ergonomisch günstige Handhabung der Nadel 10 ermöglicht. Beispielsweise kann der Querschnitt kreisrund sein, wie dies in Figur 2 dargestellt ist, oder eine abgeflachte ovale Querschnittsform aufweisen, deren Längsachse in der Krümmungsebene der Nadel 10 oder senkrecht zu dieser Krümmungsebene verlaufen kann. Der Griff 16 weist eine Aufnahme 18 auf, die sich über seine gesamte axiale Länge erstreckt und dem kreisbogenförmigen Verlauf der Nadel 10 bzw. deren Endabschnitts 14 folgt. Die Aufnahme 18 hat die Form einer radialen Nut, deren Breite so auf den Außendurchmesser der Nadel 10 abgestimmt ist, daß die Nadel 10 mit ihrem Endabschnitt 14 mit geringem Spiel in die Aufnahme 18 eingelegt werden kann. Die radiale Tiefe der Aufnahme 18 ist so gewahlt, daß die eingelegte Nadel 10 mit ihrem Endabschnitt 14 sich im wesentlichen in der Mittelachse des Griffes 16 befindet, wie dies aus Figur 2 deutlich wird.

In den Griff 16 ist eine Klemmschraube 20 eingedreht, die senkrecht zu der Aufnahme 18 diametral in den Griff 16 hineinführt. Der Klemmschraube 20 diametral gegenüber ist die Aufnahme 18 durch eine in Längsrichtung durchlaufende Längskerbe 22 erweitert. Die Nadel 10 wird mit ihrem proximalen Endabschnitt 14 seitlich in die Aufnahme 18 des Griffes 16 eingelegt. Befindet sich der Endabschnitt 14 im Grund der Aufnahme 18, so wird die Klemmschraube 20 in den Griff 16 eingedreht, so daß sie den Endabschnitt 14 in die Längskerbe 22 drückt. Aufgrund der kreisbogenförmigen Krümmung der Nadel 10 und ihres Endabschnittes 14 und der entsprechenden kreisbogenförmigen Krümmung des Griffes 16 und seiner Aufnahme 18 ist dadurch die Nadel 10 unverdrehbar in dem Griff 16 gehalten. Vorzugsweise weist die Nadel 10 in ihrem Endabschnitt 14 außerdem einen Einstich 24 in ihrem Umfang auf, in welchen die Klemmschraube 20 mit ihrer Spitze eingreift. Durch den Eingriff der Klemmschraube 20 in den Einstich 24 werden der Endabschnitt 14 und damit die gesamte Nadel 10 axial unverschiebbar in dem Griff 16 festgelegt. Allein mittels der Klemmschraube 20 kann auf diese Weise der Griff 16 mit der Nadel 10 lösbar und sowohl in axialer Richtung als auch in Rotationsrichtung formschlüssig verbunden werden.

An dem proximalen hinteren Ende der Nadel 10, d.h. an dem aus dem Griff 16 nach hinten herausragenden Ende des Endabschnittes 14 wird ein Band 26 lösbar angekuppelt. Das Band 26 kann aus einem nichtresorbierbaren Nahtmaterial, z.B. aus Polypropylen, oder teilweise oder ganz aus einem resorbierbaren Nahtmaterial geflochten sein. Das Band 26 weist somit eine Dehnbarkeit sowohl in seiner Längsrichtung als auch in seiner Breite auf. An den Rändern des Bandes 26 ist sein Geflecht offen, so daß die Fäden stachelartig nach außen stehen. Das Band 26 ist mit einer glatten Kunststoffhülle 28 umschlossen, die in der Mitte der axialen Länge des Bandes 26 geteilt ist und sich überlappt oder mit einer Sollbruchnaht trennbar ist.

Um das Band 26 mit der Hülle 28 lösbar an den Nadeln 10 anzukuppeln sind die beiden Enden des Bandes 26 und der Hülle 28 jeweils in einen Adapter 30 eingesetzt, wie dies insbesondere in Figur 3 gezeigt ist. Der Adapter 30 hat die Form einer rohrförmigen Hülse, in deren proximales Ende das Band 26 mit der Hülle 28 eingesetzt und befestigt ist. Hierzu kann das Band 26 mit der Hülle 28 in den aus Kunststoff bestehenden Adapter 30 eingeklebt oder eingeschweißt sein. Der Außendurchmesser des Adapters 30 stimmt mit dem Außendurchmesser der Nadel 10 und deren Endabschnitts 14 überein. Mit seinem distalen vorderen Ende wird der Adapter 30 auf einen Konnektor 32 geschoben, der an der proximalen Endstirnfläche des Endabschnittes 14 als koaxialer Vorsprung ausgebildet ist. Der Außendurchmesser des Konnektors 32 ist gegenüber dem Durchmesser des Endabschnittes 14 reduziert und stimmt mit dem Innendurchmesser des Adapters 30 überein. An dem freien Ende des Konnektors 32 ist ein radial abstehender Zapfen 34 angeformt. Beim Einschieben des Konnektors 32 in den Adapter 30 gleitet der Zapfen 34 in einen axialen Längsschlitz 36 des Adapters 30, der an seinem inneren Ende in einen in Umfangsrichtung weisenden Querschlitz 38 ausläuft. Sobald der Zapfen 34 den Querschlitz 38 erreicht hat, wird der Konnektor 32 gegen den Adapter 30 verdreht, so daß der Zapfen 34 in den Querschlitz 38 gelangt. Auf diese Weise ist der Adapter 30 mit dem Konnektor 32 und damit das Band 26 und seine Hülle 28 mit der Nadel 10 nach Art einer Bajonett-Verriegelung lösbar gekuppelt.

Das Band 26 mit seiner Hülle 28 und den an beiden Enden befestigten Adaptern 30 ist ein vollständig aus Kunststoff hergestellter preisgünstiger Einmal-Artikel. Die Nadel 10 und der Griff 16 sind dagegen mehrfach verwendbar und sterilisierbare Artikel.

Zum Einziehen des Bandes 26 wird der Griff 16 auf dem Endabschnitt 14 der Nadel 10 befestigt und ein Band 26 mit Hülle 28 wird mittels des Adapters 30 hinter dem Griff 16 mit dem proximalen Ende der Nadel 10 konnektiert. Nach einer medianen Kolpotomie wird die Nadel 10 eingeführt. Die Nadel 10 wird dann seitlich an der Urethra vorbei an der Hinterwand des Schambeins 40 entlanggeführt, wie dies schematisch in Figur 4 dargestellt ist. Dabei folgt die Nadel 10 aufgrund ihrer Krümmung dem gekrümmten Verlauf der Hinterwand des Schambeins 40, wobei eine ergonomisch günstige Führung und Vorschubbewegung der Nadel 10 dadurch möglich ist, daß die Nadel 10 mittels des Griffes 16 über eine große axiale Länge gefaßt und bogenförmig geführt werden kann. Sobald die Spitze 12 die Bauchmuskulatur erreicht hat, wird diese durch axialen Druck auf die Nadel 10 mittels des Griffes 16 perforiert, so daß die Spitze 12 durch die Bauchdecke austritt. Sobald die Spitze 12 soweit aus der Bauchdecke herausgetreten ist, daß die Nadel zuverlässig gefaßt werden kann, wird der Griff 16 nach Lösen der Klemmschraube 20 von dem Endabschnitt 14 der Nadel 10 abgenommen. Die Nadel 10 kann nun an ihrem distalen Ende erfasst und vollständig durchgezogen werden, so daß zunächst der Endabschnitt 14 der Nadel 10 und anschließend das Band 26 durch das Gewebe gezogen werden, bis das Band 26 mit dem Adapter 30 durch die Bauchdecke herausgezogen sind. Da der Adapter 30 sich mit gleichem Außendurchmesser an dem Endabschnitt 14 anschließt, behindert die Kupplung des Bandes 26 an die Nadel 10 das Durchziehen nicht. Die das Band 26. umschließende Hülle 28 gewährleistet, daß das Band 26 unbehindert durch das Gewebe gezogen werden kann, ohne daß dieses Gewebe zusätzlich geschädigt wird.

Sobald auf diese Weise das eine Ende des Bandes 26 eingezogen ist, wird die Konnektierung des Bandes 26 an der Nadel 10 mittels des Adapters 30 und des Konnektors 32 gelöst und die gleiche Nadel 10 oder eine weitere Nadel wird eingestochen und auf der anderen Seite der Urethra durchgestoßen. Dann wird diese Nadel 10 in gleicher Weise mit dem anderen Ende des Bandes 26 konnektiert. Nun wird in entsprechender Weise das andere Ende des Bandes 26 mittels der Nadel 10 eingezogen, wobei das Band auf der anderen Seite der Urethra vorbeigeführt wird. Ist auf diese Weise auch das zweite Ende des Bandes 26 durch die Bauchdecke herausgezogen, so wird die Nadel 10 auch von diesem zweiten Ende des Bandes 26 getrennt. Nach einer exakten Positionierung des Bandes werden bei konventioneller Vorgehensweise die Adapter 30 abgetrennt und die Hüllen abgezogen und das Band im Bereich der Subcutis abgeschnitten. Falls dabei das Band beim Abziehen der Hüllen nicht in seiner gewünschten Position gehalten werden kann, kann eine Vorrichtung zur definierten Positionierung und Fixation der Bandenden nach der Penetration der Bauchdecken bei der Extraktion der Bandhüllen verwendet werden. Dabei handelt es sich um eine Vorrichtung, welche sich mit einer Bodenplatte auf den Beckenknochen der Patientin abstützt und eine Verbindung der Adapter 30 der Bandenden an ein gegenüber der Bodenplatte verschiebbares Gestänge erlaubt. Die Bandenden werden mittels der Adapter 30 an dem Gestänge befestigt und durch Verschieben des Geständes wird das Band 26 in seiner exakten Positionierung gehalten. Dann werden die Bandhüllen 28 durchtrennt und abgezogen, während das Band 26 positioniert gehalten wird und sich im Gewebe verankern kann. Dann werden die Bandenden im Bereich der Subcutis abgeschnitten. Dadurch kommen die Ränder des Bandes 26 frei und können sich in dem Gewebe verhaken, so daß das Band 26 in dem Gewebe fixiert ist.

Wie die Figuren 4 und 5 zeigen, ist die distale Spitze 12 der Nadel 10 so geformt, daß ihr Scheitelpunkt 42 nicht mit der kreisbogenförmigen axialen Mittellinie 44 der Nadel 10 zusammenfällt, die in den Figuren 4 und 5 punktiert eingezeichnet ist. Die Spitze 12 hat vielmehr die Form eines schrägen Kreiskegels, dessen Scheitelpunkt 42 in der durch die Mittellinie 44 verlaufenden Krümmungsebene der Nadel 10 liegt, jedoch gegen die Mittellinie 44 in Richtung auf den Krümmungsmittelpunkt nach innen versetzt angeordnet ist. Vorzugsweise fällt der Scheitelpunkt 42 mit der Mantelfläche der Nadel 10 zusammen. Vorzugsweise ist die Spitze 12 an ihrem Scheitelpunkt 42 so angeschliffen, daß eine der Krümmungsmitte zugewandte Kufe 46 gebildet wird. Durch das Anschleifen der Kufe 46 wird der scharfe penetrierende Scheitelpunkt 42 der Spitze 12 wiederum von der Mantelfläche der Nadel 10 beabstandet. Das distale Ende der Nadel 10 ist zusätzlich auch mit einem kleineren Krümmungsradius als der Krümmungsradius der Nadel 10 stärker nach innen gebogen. Dies ist in Fig. 4 dargestellt, in welcher der kreisbogenförmige Verlauf der Nadel 10 und ihrer Mittellinie 44 strichliert bzw. strichpunktiert über die Spitze 12 hinaus verlängert eingezeichnet sind. Es ist in Figur 4 erkennbar, daß durch diese stärkere Krümmung der Scheitelpunkt 42 der Spitze 12 gegen den Mantel der Nadel 10 nach innen auf den Krümmungsmittelpunkt hin versetzt ist.

Wird die Nadel 10 eingestochen, so läuft die Spitze 12 mit ihrem Scheitelpunkt 42 an der Hinterwand des Schambeins 40 entlang, wie dies in Fig. 4 dargestellt ist. Da die Spitze 12 der Schambeinrückwand zugewandt ist, wird die Gefahr einer Beschädigung der Harnblase vermieden. Falls die Spitze 12 beim Vorschieben der Nadel 10 mit der Harnblase in Berührung kommen sollte, drückt die Außenfläche 48 der. Spitze 12 die Harnblase wie ein stumpfer Schild weg, so daß eine Verletzung der Harnblase durch den scharfen Scheitelpunkt 42 der Spitze 12 vermieden wird. Durch die angeschliffene Kufe 46 wird andererseits vermieden, daß die Spitze 12 beim Entlanggleiten an der Rückwand des Schambeins 40 die Knochenhaut verletzt. Die Spitze 12 sitzt mit der Kufe 46 auf dem Schambein 40 auf, ohne daß der scharfe Scheitelpunkt 42 mit der Knochenhaut des Schambeins 42 in Berührung kommen kann. Da die Spitze 12 gegenüber dem kreisbogenförmigen Verlauf der Nadel 10 stärker nach innen gekrümmt ist, wie dies in Fig. 4 gezeigt ist, wird hierdurch das Aufsitzen der Kufe 46 auf der Rückwand des Schambeins 40 zusätzlich begünstigt, wenn die Nadel 10 auf einer kreisbogenförmigen Bahn, die der Krümmung der Nadel 10 entspricht, entlang dem Schambein 40 vorgeschoben wird. Die asymmetrische Ausbildung der Spitze 12 in Bezug auf die kreisbogenförmige Mittellinie 44 der Nadel 10 hat zusätzlich den Effekt, daß beim Vorschieben der Nadel 10 auf einer ihrer Mittellinie 44 entsprechenden Kreisbogenbahn ein asymmetrischer Druck des penetrierten Gewebes auf die Spitze 12 wirkt, der diese Spitze leicht gegen das Schambein 40 drückt, so daß sich eine gewisse zwangsläufige Führung der Spitze 12 an der rückseitigen Oberfläche des Schambeins 40 ergibt. Dadurch kann insgesamt beim Einführen der Nadel 10 mit deren Spitze 12 das Schambein 40 ertastet werden, so daß eine optimale und einfache Führung der Nadel 10 möglich ist, ohne daß die Gefahr einer Traumatisierung der Harnblase oder der Knochenhaut des Schambeins 40 besteht.

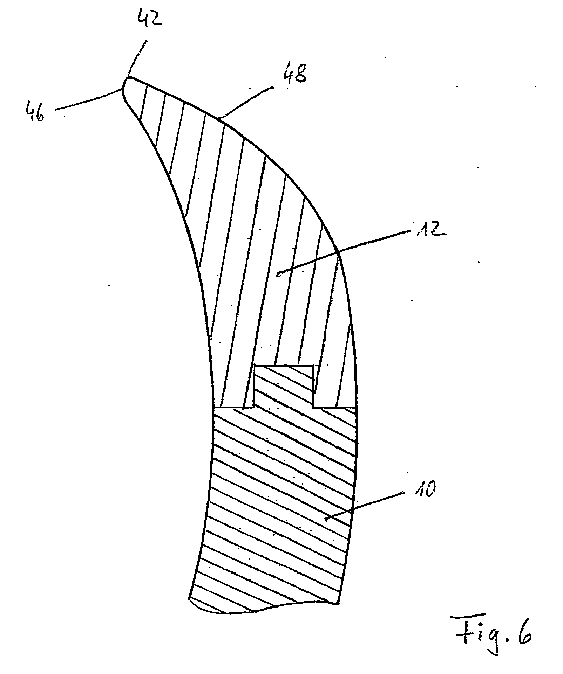

Fig. 6 zeigt eine Abwandlung der Erfindung, bei welcher die Nadel 10 und ihre Spitze 12 nicht einstückig aus Stahl hergestellt sind. Die Spitze 12 ist vielmehr aus einem geeigneten Kunststoff gefertigt und auf das distale Ende der aus Stahl bestehenden Nadel 10 aufgesetzt. Die Spitze 12 kann in einer an sich bekannten Technik an dem distalen Ende der Nadel 10 befestigt werden, z. B. aufgepreßt, aufgeklebt oder aufgespritzt.

Es ist ohne weiteres ersichtlich, daß die erfindungsgemäße Ausbildung der Spitze 12 der Nadel 10 auch bei anderen Ausführungen der Nadel und deren Konnektierung mit dem Band 26 verwendet werden kann. Ebenso ist die Ausbildung der Spitze 12 der Nadel 10 nicht beschränkt auf Nadeln, mit welchen ein Band 26 eingezogen wird, sondern kann auch bei anderen Instrumentarien verwendet werden, bei welchen mittels der Nadel 10 zunächst ein Hilfsmittel eingezogen wird, das anschließend zum Einziehen des Bandes verwendet wird. Grundsätzlich kann die erfindungsgemäße Ausgestaltung der Nadel 10 mit der Spitze 12 für alle Anwendungen eingesetzt werden, bei welchen eine Nadel unter Abtastung der Rückwand des Schambeins eingestochen wird.

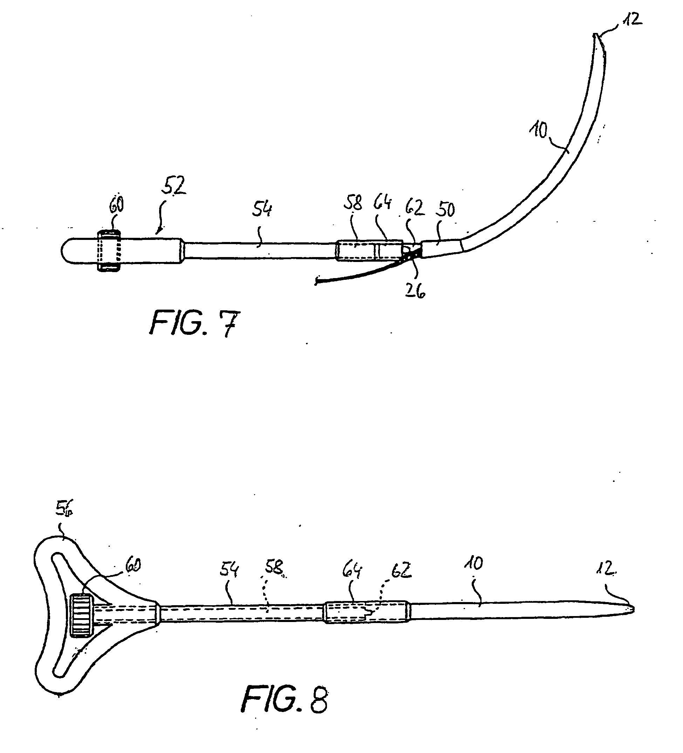

Ein Beispiel für eine andere Ausgestaltung ist in den Fig. 7 und 8 dargestellt. In diesem Ausführungsbeispiel ist das Band 26 mittels einer Tülle 50 außen am Umfang des proximalen Endes der gebogenen Nadel 10 befestigt. Zum Einstechender Nadel 10 ist an deren proximalem Ende ein Griff 52 angebracht, der aus einem die Nadel 10 axial verlängernden Schaft 54 mit einem Griffflügel 56 besteht. In dem Schaft 54 ist eine Gewindestange 58 geführt, die mittels eines Rändelkopfes 60 in eine koaxiale Gewindebohrung 62 des proximalen Endes der Nadel 10 einschraubbar ist. Die Konnektierung wird durch eine Überwurfhülse 64 abgedeckt. Die Ausbildung des Griffes 52 und seiner Konnektierung am proximalen Ende der Nadel 10 entsprechen hierbei der US 5,899,909.

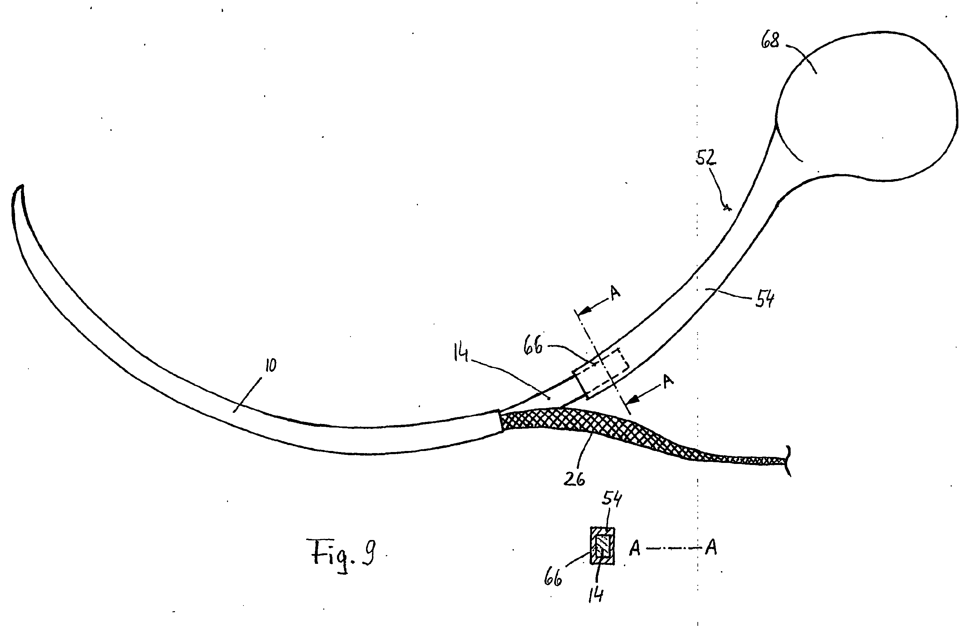

In Fig. 9 ist eine weitere Abwandlung des Instrumentariums dargestellt. Hierbei ist das Band 26 in ähnlicher Weise außen am Umfang des proximalen Endes 14 der gebogenen Nadel 10 befestigt, wie dies z. B. in Figur 7 dargestellt ist. Das proximale Ende 14 der Nadel 10 weist einen unrunden Querschnitt auf, z. B. einen rechteckigen Querschnitt, wie dies in dem Schnitt A-A in Fig. 9 dargestellt ist. Auf dieses proximale Ende der Nadel 10 wird ein Griff 52 axial aufgesteckt. Hierzu weist der Schaft 54 des Griffes 52 an seinem vorderen Ende eine sacklochartige Aufnahme 66 auf, deren Innenquerschnitt dem unrunden Außenquerschnitt des proximalen Endes 14 der Nadel 10 entspricht. Aufgrund des unrunden Querschnittes des proximalen Endes der Nadel 10 und der Aufnahme 66 wird die Nadel 10 unverdrehbar mit dem Griff 52 verbunden. Da die Aufnahme 66 als Sackloch ausgebildet ist, wird die Nadel 10 in dem Schaft 54 des Griffes 52 axial abgestützt, wenn die Nadel 10 mittels des Griffes 52 eingestochen wird. Da mit dem Griff 52 im Wesentlichen nur eine axiale Vorschubkraft auf die Nadel 10 ausgeübt wird, genügt eine geringe Haltekraft, um die Nadel 10 in der Aufnahme 66 zu halten. Diese Haltekraft kann durch eine dem Fachmann geläufige Klemmeinrichtung bewirkt werden, z. B. durch eine in die Aufnahme 66 eingesetzte Feder, durch eine Rastkugel oder einen Rastzapfen. In dem Ausführungsbeispiel der Figur 9 ist der Schaft 54 des Griffes 52 in seiner Längsrichtung gekrümmt. Vorzugsweise weist dabei der Schaft 54 denselben Krümmungsradius wie die Nadel 10 auf, so dass der Schaft 54 beim Einstechen und Durchstoßen der Nadel 10 der Verlängerung des Bahnberlaufs der Nadel 10 folgt. Dadurch ist eine optimale Kraftübertragung von dem Griff 52 auf die Nadel 10 möglich, wenn diese Nadel 10 auf einer gekrümmten Bahn durch das Gewebe gestoßen wird. Am proximalen Ende des Schaftes 54 ist eine Verdickung 68 angeformt, die die Form einer Kugel oder dergleichen hat und gewährleistet, dass der Griff 52 ergonomisch günstig in der Hand des Operateurs liegt, wenn dieser die Nadel einsticht. Vorzugsweise ist der Querschnitt des proximalen Endabschnittes 14 gegenüber dem Querschnitt der Nadel 10 ezwas verkleinert. Dadurch können das Band 26 und dessen Hülle außen auf dem Umfang des Endabschnittes 14 befestigt werden, ohne den Querschnitt der Nadel 10 zu verdicken und das Durchziehen der Nadel 10 durch das Gewebe zu behindern.

## Patentansprüche

1. Instrumentarium zur operativen Behandlung der Harninkontinenz der Frau mit wenigstens einer gebogenen Nadel (10), deren distales Ende als Spitze (12) zum Penetrieren des Gewebes ausgebildet ist und an deren proximalem Ende ein implantierbares Band (26) befestigbar ist, welches mittels der Nadel (10) als Schleife um die Urethra gelegt wird,
**dadurch gekennzeichnet, daß** die distale Spitze (12) der Nadel (10) gegenüber der Mittelachse (44) der Nadel (10) in deren Krümmungsebene gegen die Seite des Krümmungsmittelpunktes hin versetzt ist und daß das distale Ende der Nadel (10) mit einem Krümmungsradius, der kleiner ist als der Krümmungsradius der Nadel (10), gegen die Seite des Krümmungsmittelpunktes hin gekrümmt ist.

2. Instrumentarium nach Anspruch 1,
**dadurch gekennzeichnet, daß** die Spitze (12) einen schiefen Kegel bildet, dessen Scheitelpunkt (42) auf dem Mantel der Nadel (10) in der Krümmungsebene liegt.

3. Instrumentarium nach Anspruch 2,
**dadurch gekennzeichnet,**
**daß** die Spitze (12) an ihrem Scheitelpunkt (42) zu einer Kufe (46) geschliffen ist.

4. Instrumentarium nach einem der Ansprüche 1-3,
**dadurch gekennzeichnet, daß** die Spitze (12) aus Kunststoff besteht und an dem distalen Ende der Nadel (10) angebracht ist.

5. Instrumentarium nach einem der Ansprüche 1-4,
**dadurch gekennzeichnet, daß** das Band (26) und ggf. eine das Band (26) umschließende Hülle (28) mittels einer lösbaren Kupplung (30, 32) an dem proximalen Ende der Nadel (10) befestigbar ist, und daß ein Griff (16) lösbar an der Nadel (10) anbringbar ist.

6. Instrumentarium nach Anspruch 5,
**dadurch gekennzeichnet, daß** das Band (26) und ggf. die Hülle (28) an ihren beiden Enden jeweils mit einem Adapter (30) fest verbunden sind, welcher mit einem an dem proximalem Ende der Nadel (10) ausgebildeten Konnektor (32) kuppelbar ist.

7. Instrumentarium nach Anspruch 5 oder 6,
**dadurch gekennzeichnet, daß** sowohl der Konnektor (32) als auch der Adapter (30) mit dem daran befestigten Band (26) und ggf. der Hülle (28) einen Außendurchmesser aufweisen, der nicht größer ist als der Außendurchmesser der Nadel (10).

8. Instrumentarium nach Anspruch 6 oder 7,
**dadurch gekennzeichnet, daß** der Adapter (30) und der Konnektor (32) koaxial ineinander greifen und formschlüssig miteinander verbindbar sind, wobei das Band (26) und ggf. die Hülle (28) an dem proximalen Endabschnitt des Adapters (30) befestigt sind, welcher nicht mit dem Konnektor (32) in Eingriff kommt.

9. Instrumentarium nach Anspruch 8,
**dadurch gekennzeichnet, daß** der Konnektor (32) und der Adapter (30) nach Art eines Bajonettverschlusses (34,36,38) miteinander verbindbar sind.

10. Instrumentarium nach einem der Ansprüche 1 bis 9,
**dadurch gekennzeichnet, daß** die Nadel (10) einen proximalen Endabschnitt (14) aufweist und daß ein Griff (16) lösbar auf diesen Endabschnitt (14) in der Weise aufsetzbar ist, daß die Nadel (10) mit ihrem Endabschnitt (14) in dem Griff (16) axial fest und unverdrehbar gehalten ist.

11. Instrumentarium nach Anspruch 10,
**dadurch gekennzeichnet, daß** der Griff (16) eine über seine axiale Länge durchgehende, sich radial öffnende Aufnahme (18) aufweist, in welche der Endabschnitt (14) einlegbar und fixierbar ist.

12. Instrumentarium nach Anspruch 11,
**dadurch gekennzeichnet,**
**daß** der Endabschnitt (14) der Nadel (10) in der Aufnahme (18) klemmbar ist.

13. Instrumentarium nach Anspruch 10,
**dadurch gekennzeichnet, dass** an dem proximalen Endabschnitt (14) der Nadel (10) ein Griff (52) konnektierbar ist, der einen die Nadel (10) axial verlängernden Schaft (54) aufweist.

14. Instrumentarium nach Anspruch 13,
**dadurch gekennzeichnet, dass** der Schaft (54) auf den einen unrunden Querschnitt aufweisenden Endabschnitt (14) aufsteckbar ist.

15. Instrumentarium nach Anspruch 13 oder 14,
**dadurch gekennzeichnet, dass** der Schaft (54) des Griffes (52) in Längsrichtung gekrümmt ist und vorzugsweise die gekrümmte Nadel (10) mit dem gleichen Krümmungsradius axial verlängert.

16. Instrumentarium nach einem der Ansprüche 5 bis 15,
**dadurch gekennzeichnet,**
**daß** das Band (26) und ggf. die Hülle (28) ein konfektionierter Einmal-Artikel und die Nadel (10) und der Griff(16) mehrfach verwendbare sterilisierbare Artikel sind.

17. Instrumentarium nach einem der vorhergehenden Ansprüche,
**dadurch gekennzeichnet,**
**daß** das Band (26) aus einem resorbierbaren Kunststoff besteht.

## Claims

1. An instrument for surgically treating female urinary incontinence, having at least a curved needle (10), the distal end of which is designed as a tip (12) to penetrate the tissue and at the proximal end of which an implantable band (26) can be attached, which is laid as a loop around the urethra by means of the needle (10),
**characterised in that** the distal tip (12) of the needle (10) is offset in relation to the centre axis (44) of the needle (10) in its plane of curvature towards the side of the centre of curvature
and **in that** the distal end of the needle (10), having a radius of curvature less than the radius of curvature of the needle (10), is curved towards the side of the centre of curvature.

2. An instrument according to Claim 1,
**characterised in that** the tip (12) forms a bevelled cone, the vertex (42) of which lies on the shell of the needle (12) in the plane of curvature.

3. An instrument according to Claim 2,
**characterised in that** the tip (12) is bevelled at its vertex (42) to form a skid (46).

4. An instrument according to one of Claims 1 to 3,
**characterised in that** the tip (12) is made from plastic and is mounted at the distal end of the needle (10).

5. An instrument according to one of Claims 1 to 4,
**characterised in that** the band (26) and where appropriate a casing (28) surrounding the band (26) can be fasted by means of a releasable coupling (30, 32) to the proximal end of the needle (10),
and **in that** a grip (16) can be detachably mounted on the needle (10).

6. An instrument according to Claim 5,
**characterised in that** the band (26) and where appropriate the casing (28), at their two ends, are in each case securely connected to an adapter (30), which can be coupled to a connector (32) provided at the proximal end of the needle (10).

7. An instrument according to Claim 5 or 6,
**characterised in that** the connector (32) as well as the adapter. (30) with the band (26) fastened thereto and possibly the casing (28) have an external diameter that is not greater than the external diameter of the needle (10).

8. An instrument according to Claim 6 or 7,
**characterised in that** the adapter (30) and the connector (32) engage coaxially and can be connect to each other in form fit manner, with the band (26) and where appropriate the casing (28) being fastened to the proximal end portion of the adapter (30), which does not come into engagement with the connector (32).

9. An instrument according to Claim 8,
**characterised in that** the connector (32) and the adapter (30) can be connected to each other in the manner of a bayonet coupling.

10. An instrument according to one of Claims 1 to 9,
**characterised in that** the needle (10) comprises a proximal end portion (14)
and **in that** a grip (16) can be detachably placed on this end portion (14) in such a manner that the needle (10) is retained by its end portion (14) in the grip (16) securely and non-twistably in the axial direction.

11. An instrument according to Claim 10,
**characterised in that** the grip (16) comprises a receptacle (18) that pierces its axial length and radially opens, into which the end portion (14) can be inserted and fixed.

12. An instrument according to Claim 11,
**characterised in that** the end portion (14) of the needle (10) can be clamped in the receptacle (18).

13. An instrument according to Claim 10,
**characterised in that** a grip (52) comprising a shaft (54) that lengthens the needle (10) in the axial direction can be connected to the proximal end portion (14) of the needle (10) .

14. An instrument according to Claim 13,
**characterised in that** the shaft (54) can be placed onto the end portion (14) having a non-circular cross section.

15. An instrument according to Claim 13 or 14,
**characterised in that** the shaft (54) of the grip (52) is curved in the longitudinal direction and preferably lengthens the curved needle (10) with the same radius of curvature in the axial direction.

16. An instrument according to one of Claims 5 to 15,
**characterised in that** the band (26) and where appropriate the casing (28) are a prefabricated disposable item and the needle (10) and the grip (16) are multi-use sterilisable items.

17. An instrument according to one of the preceding Claims,
**characterised in that** the band (26) is made from an absorbable plastic material.

## Revendications

1. Instrument de traitement chirurgical de l'incontinence urinaire chez la femme, comprenant au moins une aiguille courbe (10) dont l'extrémité distale présente la forme d'une pointe (12) pour pénétrer dans le tissu et à l'extrémité proximale de laquelle une bande implantable (26) peut être fixée pour être placée en formant une boucle autour de l'urètre au moyen de l'aiguille (10),
**caractérisé en ce que**
la pointe distale (12) de l'aiguille (10) est décalée par rapport à l'axe médian (44) de l'aiguille (10), dans le plan de courbure de celle-ci vers le côté du point médian de courbure, et l'extrémité distale de l'aiguille (10) est courbée avec un rayon de courbure inférieur au rayon de courbure de l'aiguille (10) vers le côté du point médian de courbure.

2. Instrument selon la revendication 1,
**caractérisé en ce que**
la pointe (12) forme un cône incliné dont le sommet (42) se situe sur l'enveloppe de l'aiguille (10) dans le plan de courbure.

3. Instrument selon la revendication 2,
**caractérisé en ce que**
la pointe (12) est meulée au niveau de son sommet (42) pour former un patin (46).

4. Instrument selon l'une quelconque des revendications 1 à 3,
**caractérisé en ce que**
la pointe (12) est en matière plastique et disposée à l'extrémité distale de l'aiguille (10).

5. Instrument selon l'une quelconque des revendications 1 à 4,
**caractérisé en ce que**
la bande (26) et le cas échéant une gaine (28) entourant la bande (26) peuvent être fixées à l'extrémité proximale de l'aiguille (10) au moyen d'un couplage amovible (30, 32), et une poignée (16) peut être attachée de manière amovible à l'aiguille (10).

6. Instrument selon la revendication 5,
**caractérisé en ce que**
la bande (26) et le cas échant la gaine (28) ont leurs deux extrémités respectivement solidaires d'un adaptateur (30), qui peut être couplé avec un connecteur (32) prévu à l'extrémité proximale de l'aiguille (10).

7. Instrument selon la revendication 5 ou 6,
**caractérisé en ce qu'**
aussi bien le connecteur (32) que l'adaptateur (30) avec la bande (26) fixée à celui-ci et le cas échéant la gaine (28) présentent un diamètre extérieur qui n'est pas plus grand que le diamètre extérieur de l'aiguille (10).

8. Instrument selon la revendication 6 ou 7,
**caractérisé en ce que**
l'adaptateur (30) et le connecteur (32) s'engrènent coaxialement l'un dans l'autre et peuvent être reliés l'un à l'autre par complémentarité de formes, la bande (26) et le cas échéant la gaine (28) étant fixées au segment final proximal de l'adaptateur (30) qui n'est pas engrené avec le connecteur (32).

9. Instrument selon la revendication 8,
**caractérisé en ce que**
le connecteur (32) et l'adaptateur (30) peuvent être reliés l'un à l'autre en forme d'une fermeture à baïonnette (34, 36, 38).

10. Instrument selon l'une quelconque des revendications 1 à 9,
**caractérisé en ce que**
l'aiguille (10) présente un segment final proximal (14), et une poignée (16) peut être placée de façon amovible sur ce segment final (14), de sorte que l'aiguille (10) est maintenue par son segment final (14) dans la poignée (16) de façon fixe axialement et sans rotation.

11. Instrument selon la revendication 10,
**caractérisé en ce que**
la poignée (16) présente un logement (18) s'étendant sur sa longueur axiale et ouvert radialement, dans lequel on peut insérer et fixer le segment final (14).

12. Instrument selon la revendication 11,
**caractérisé en ce que**
le segment final (14) de l'aiguille (10) peut être serré dans le logement (18).

13. Instrument selon la revendication 10,
**caractérisé en ce qu'**
au segment final proximal (14) de l'aiguille (10) on peut connecter une poignée (52) qui présente une tige (54) prolongeant l'aiguille (10) axialement.

14. Instrument selon la revendication 13,
**caractérisé en ce que**
la tige (54) peut être enfichée sur le segment final (14) présentant une section transversale ovalisée.

15. Instrument selon la revendication 13 ou 14,
**caractérisé en ce que**
la tige (54) de la poignée (52) est courbe dans la direction longitudinale et prolonge de préférence l'aiguille courbe (10) axialement avec le même rayon de courbure.

16. Instrument selon l'une quelconque des revendications 5 à 15,
**caractérisé en ce que**
la bande (26) et le cas échéant la gaine (28) sont des articles jetables confectionnés et l'aiguille (10) ainsi que la poignée (16) sont des articles stérilisables pouvant être utilisés plusieurs fois.

17. Instrument selon l'une quelconque des revendications précédentes,
**caractérisé en ce que**
la bande (26) est fabriquée à partir d'une matière plastique résorbable.
